# FASCICULE DE BREVET EUROPEEN

(11) **EP 1 287 000 B1**
(45) Date de publication et mention de la délivrance du brevet: **12.10.2005**
(21) Numéro de dépôt: 01940638.8
(22) Date de dépôt: 30.05.2001
(51) Int. Cl.: C07D 493/04

(54) **PROCEDE DE PURIFICATION D'UNE COMPOSITION CONTENANT AU MOINS UN PRODUIT DE DESHYDRATATION INTERNE D'UN SUCRE HYDROGENE**
VERFAHREN ZUR REINIGUNG EINER MINDESTENS EIN INNERES DEHYDRATATIONSPRODUKT EINES HYDRIERTEN ZUCKERS ENTHALTENDEN MISCHUNG
METHOD FOR PURIFYING A COMPOSITION CONTAINING AT LEAST AN INTERNAL DEHYDRATION PRODUCT FOR A HYDROGENATED SUGAR

(30) Priorité: 09.06.2000 FR 0007463
(43) Date de publication de la demande: 05.03.2003
(73) Titulaire: Roquette Frères, 62136 Lestrem (FR)
(72) Inventeur: FLECHE, Guy, F-59190 Hazebrouck (FR); FUERTES, Patrick, F-59130 Lambersart (FR); TAMION, Rodolphe, F-62157 Allouagne (FR); WYART, Hervé, F-62149 Cuinchy (FR)
(74) Mandataire: Boulinguiez, Didier
(86) Numéro de dépôt international: PCT/FR2001/001676
(87) Numéro de publication internationale: WO 2001/094352

(56) Documents cités:
- EP-A- 0 052 295
- EP-A- 0 315 334
- WO-A-00/14081
- WO-A-99/54129
- GB-A- 613 444
- US-A- 4 564 692
- PATENT ABSTRACTS OF JAPAN vol. 1998, no. 08, 30 juin 1998 (1998-06-30) & JP 10 059976 A (DAINIPPON INK &CHEM INC), 3 mars 1998 (1998-03-03)
- PATENT ABSTRACTS OF JAPAN vol. 004, no. 124 (C-023), 2 septembre 1980 (1980-09-02) & JP 55 079383 A (TOWA KASEI KOGYO KK), 14 juin 1980 (1980-06-14)

## Description

La présente invention a pour objet un nouveau procédé de purification d'une composition contenant au moins un produit de déshydratation interne d'un sucre hydrogéné.

La composition purifiée ainsi obtenue est utilisable dans la préparation de produits ou mélanges polymériques ou non, biodégradables ou non, destinés en particulier aux industries chimiques, pharmaceutiques, cosmétologiques ou alimentaires.

Enfin, le produit susceptible d'être obtenu selon ledit procédé, est une composition du genre en question présentant des caractéristiques particulières en termes de pureté et de teneur en certaines impuretés.

Par « sucre hydrogéné » au sens de la présente invention, on entend en particulier :
- les hexitols tels que, par exemple, le sorbitol, le mannitol, l'iditol et le galactitol,
- les pentitols tels que, par exemple, l'arabitol, le ribitol et le xylitol, et
- les tétritols tels que, par exemple, l'érythritol.

Par « produit de déshydratation interne », on entend tout produit résultant, d'une manière quelconque, en une ou plusieurs étapes, de l'enlèvement d'une ou de plusieurs molécules d'eau au niveau de la structure interne originelle d'un sucre hydrogéné tel que ceux mentionnés ci-avant.

Il peut s'agir avantageusement de produits de déshydratation interne d'hexitols, en particulier de dianhydro hexitols ou « isohexides » comme l'isosorbide (1,4 - 3,6 dianhydro sorbitol), l'isomannide (1,4 - 3,6 dianhydro mannitol) ou l'isoidide (1,4 - 3,6 dianhydro iditol).

Parmi ces sucres hydrogénés doublement déshydratés, l'isosorbide est aujourd'hui celui pour lequel on développe, pour le moins on envisage, le plus d'applications industrielles. Celles-ci concernent notamment :
- la préparation de 2-nitrate, 5-nitrate ou 2,5 dinitrate d'isosorbide, utiles dans le traitement thérapeutique de maladies, notamment cardiaques et/ou vasculaires - comme décrit dans le brevet US 4,371,703.
- la préparation de dérivés alcoylés, en particulier diméthylés, de l'isosorbide, utiles notamment comme solvants dans le cadre de la préparation de compositions pharmaceutiques ou cosmétologiques (brevet US 4,082,881), voire comme principes actifs de compositions d'hygiène buccale (brevet EP 315 334).
- la préparation d'articles à base d'alcool polyvinylique (brevet US 4,529,666), de polyuréthannes (brevet US 4,383,051), ou de polyesters contenant également des unités monomères de type « téréphthaloyle » (brevets US 3,223,752 et US 6,025,061),
- la préparation de polycondensats biodégradables (brevet WO 99/45 054),
- la préparation de laques aqueuses (brevet US 4,418,174) ou de compositions à action recouvrante et/ou colorante de surfaces (brevet US 5,766,679).

Pour la majorité des applications précitées de l'isosorbide et autres produits de déshydratation interne de sucres hydrogénés, en particulier des autres isohexides, il est généralement requis d'appliquer un traitement de purification aux compositions résultant directement de l'étape de déshydratation proprement dite. Ceci, du fait notamment que tout sucre hydrogéné soumis à une telle étape (par exemple le sorbitol) est susceptible, lors de ladite étape, d'être transformé, outre en le produit de déshydratation recherché (par exemple l'isosorbide) en divers co-produits tels que :
- des isomères dudit produit recherché, par exemple des isomères de l'isosorbide tels que l'isomannide et l'isoidide,
- des produits moins déshydratés que le produit recherché ou que ses isomères, par exemple du sorbitan, du mannitan ou de l'iditan,
- des dérivés résultant de l'oxydation ou plus généralement de la dégradation des produits susmentionnés, ces dérivés pouvant inclure, par exemple lorsque le produit recherché est l'isosorbide, des co-produits de type déoxy monoanhydro hexitols, monoanhydro pentitols, monoanhydro tétritols, anhydro hexoses, hydroxyméthyl furfural, ou glycérine,
- des dérivés résultant de la polymérisation des produits susmentionnés, et/ou
- des espèces fortement colorées, de nature mal définies.

Il convient de rappeler que d'une manière générale, tout ou partie de ces différentes catégories de co-produits ou impuretés sont générées peu ou prou lors de l'étape de déshydratation proprement dite du sucre hydrogéné et ce, indépendamment des conditions et précautions mises en oeuvre en pratique lors de ladite étape, et par exemple indépendamment :
- de la nature et de la forme de présentation du catalyseur acide de déshydratation utilisé (acide minéral, acide organique, résine cationique, ...), ou
- de la quantité d'eau ou de solvant(s) organique(s) dans le milieu réactionnel initial, ou
- de la pureté de la composition de sucre hydrogéné, par exemple de sorbitol, utilisée comme matière première.

Différentes technologies ont été préconisées aux fins d'obtenir des compositions issues de ladite étape de déshydratation, par exemple des compositions d'isohexide(s), qui soient améliorées en termes de pureté et ce, de manière « directe » en jouant sur les conditions réactionnelles lors de ladite étape et/ou de manière « indirecte » en appliquant un ou plusieurs traitement(s) de purification après ladite étape.

A titre d'exemple, le brevet GB 613,444 décrit l'obtention, par déshydratation en milieu eau / xylène, d'une composition d'isosorbide qui est ensuite soumise à un traitement de distillation puis de recristallisation dans un mélange alcool / éther.

Le brevet EP 52 295 (ou CA 1 195 687) décrit la synthèse de produits de déshydratation de sucres hydrogénés par déshydratation à 100-160°C et en présence de catalyseurs hétérogènes fortement acides pouvant consister en résines échangeuses d'ions fortement acides.

Après élimination dudit catalyseur, les auteurs envisagent, en toute généralité, la purification du milieu réactionnel par l'un quelconque des moyens suivants : charbon actif ou résines échangeuses d'ions ou cristallisation fractionnée, distillation ou chromatographie sur colonne. Seul l'EXEMPLE 10 dudit brevet décrit une étape de purification et ce, par le seul traitement sur charbon actif du milieu réactionnel préalablement débarrassé de son catalyseur.

Un traitement de purification associant distillation et recristallisation dans un alcool aliphatique inférieur (éthanol, méthanol) est également recommandé récemment dans le brevet WO OO/14081. Ce document indique par ailleurs que dans le cas où la distillation est la seule étape de purification envisagée, il est avantageux de conduire ladite étape en présence de borohydrure de sodium.

D'autres auteurs ont également préconisé que l'étape de distillation soit menée en présence d'un composé boré, en particulier d'acide borique ou d'une résine anionique préalablement chargée en ions borate, comme décrit dans le brevet US 3,160,641.

Les brevets US 4,408,061 et EP 323,994 envisagent la mise en oeuvre de catalyseurs de déshydratation particuliers (respectivement un halogénure d'hydrogène gazeux et du fluorure d'hydrogène liquide), associés avantageusement à des acides carboxyliques comme co-catalyseurs puis la distillation des compositions brutes d'isosorbide ou d'isomannide ainsi obtenues.

Le brevet US 4,564, 692 mentionne sans aucunement la détailler, la prépurification sur « échangeurs d'ions et/ou charbon actif » de compositions d'isosorbide ou d'isomannide puis, après concentration par évaporation et ensemencement de cristaux de l'isohexide recherché, la cristallisation dans l'eau de celui-ci.

Le brevet EP 380,402 revendique pour sa part la déshydratation de sucres hydrogénés en présence d'hydrogène sous pression et de catalyseurs particuliers à base d'une association entre le cuivre et un métal noble du Groupe VIII ou de l'or. Ces conditions sont présentées comme permettant de diminuer significativement la formation d'impuretés de nature polymèrique lors de l'étape de déshydratation proprement dite.

Plus récemment, il a été décrit dans le brevet EP 915,091, la possibilité de diminuer encore avantageusement la génèse de tels polymères indésirables et ce, par mise en oeuvre de catalyseurs d'hydrogénation stables aux acides lors de l'étape de déshydratation.

Il résulte de ce qui précède que l'obtention de compositions de haute pureté à base d'isohexide(s) ou d'autre(s) sucre(s) hydrogéné(s) déshydraté(s) impose généralement la mise en oeuvre, au moins à un moment donné, de moyens qui sont, pour le moins :
- Soit coûteux comme l'hydrogène associé aux catalyseurs et cocatalyseurs d'hydrogénation,
- Soit potentiellement dangereux pour l'homme et l'environnement, en tous cas d'usage très réglementé, comme les solvants organiques,
- Soit peu efficaces en termes de rendements comme la technologie de cristallisation dans l'eau.

En outre, les brevets précités ne s'intéressent généralement pas au problème de stabilité dans le temps des compositions purifiées obtenues, y compris de celles qui ont été plus ou moins décolorées lors du traitement de purification.

Selon la Demanderesse, il n'a d'ailleurs pas été possible jusqu'alors dans la pratique industrielle, de préparer efficacement des compositions, par exemple d'isosorbide, présentant simultanément une pureté d'au moins 98,5 % (sec/sec), une couleur blanche (en poudre) ou incolores (en solution) et une bonne stabilité, sans faire appel successivement à une étape de distillation puis à une étape de cristallisation en milieu solvant organique.
En effet, la voie alternative consistant en une simple distillation du milieu issu de l'étape de déshydratation et sa transformation, par refroidissement, en une composition de type « massé » ne permet pas d'atteindre des résultats satisfaisants en termes de purification, notamment de coloration (couleur jaune à brune) et de stabilité. La mise en oeuvre, avant évaporation, d'une étape complémentaire de traitement sur charbon actif granulaire d'un distillat préalablement remis en solution, permet d'obtenir une composition améliorée en termes de couleur mais aucunement en termes de pureté. De plus, une telle composition présente une stabilité dans le temps relativement limitée.
La Société Demanderesse a trouvé, après de nombreuses recherches, qu'il était possible d'obtenir des compositions à base d'isohexide(s), et autres sucres hydrogénés déshydratés, de haute pureté (y compris supérieure ou égale à 99,5 %) et de stabilité améliorée, sans faire appel obligatoirement à une étape de cristallisation en milieu solvant et ce, en mettant en oeuvre, dans un ordre particulier, au moins une étape de traitement de décoloration et au moins une étape d'échange ionique.
De manière plus précise, la présente invention a pour objet un procédé de purification d'une composition contenant au moins un produit de déshydratation interne d'un sucre hydrogéné, caractérisé en ce qu'il comprend :
a) une étape éventuelle au cours de laquelle ladite composition est traitée, après remise en solution ou non, avec au moins un moyen de décoloration,
b) une étape subséquente au cours de laquelle la composition, éventuellement traitée avec un moyen de décoloration, est traitée avec au moins un moyen d'échange ionique, et,
c) une étape subséquente au cours de laquelle la composition résultante est traitée avec au moins un moyen de décoloration.

Selon une variante avantageuse dudit procédé, celui-ci est caractérisé en ce qu'il comprend :
a) une étape au cours de laquelle la composition, remise en solution ou non, est traitée avec du charbon actif,
b) une étape subséquente au cours de laquelle la composition résultante est traitée avec au moins un moyen d'échange ionique,
c) une étape subséquente au cours de laquelle la composition résultante est traitée avec du charbon actif.

De manière encore plus préférentielle, le charbon actif utilisé :
- lors de l'étape a) est sous forme granulaire,
- lors de l'étape c) est sous forme pulvérulente.

Selon une autre variante, le moyen d'échange ionique utilisé lors de l'étape b) comprend au moins une résine anionique et au moins une résine cationique. De préférence, ce moyen est composé d'un lit mixte de résines anionique(s) et cationique(s) ou d'une succession de résines cationique(s) puis anionique(s) ou d'une succession de résine(s) anionique(s) puis cationique(s). De manière très avantageuse, les résines utilisées sont des résines cationique(s) et anionique(s) fortes.

Selon un autre mode du procédé selon l'invention, celui-ci comprend une étape d) au cours de laquelle la composition résultant de l'étape c) est soumise, après filtration éventuelle, à un traitement de concentration en vue d'obtenir un « massé » puis éventuellement de cristallisation , en particulier en milieu solvant organique et/ou de séchage.

Les compositions soumises au procédé de purification selon l'invention contiennent au moins un produit de déshydratation interne d'un sucre hydrogéné tel que défini ci-avant, la déshydratation pouvant être totale ou partielle.

En général, lesdites compositions contiennent un mélange de plusieurs de ces produits dans lequel prédomine pondéralement l'un d'eux. Le but premier du procédé de purification selon l'invention est de conférer à ces compositions des propriétés améliorées en termes de stabilité et de coloration et ce tout en préservant, éventuellement en augmentant, la proportion pondérale de ce sucre hydrogéné particulier en regard de la totalité des autres espèces contenues dans la matière sèche (« MS ») desdites compositions.

Ces compositions peuvent être obtenues selon l'une quelconque des étapes de déshydratation puis, éventuellement, de distillation décrites dans l'un quelconque des brevets précités. Le procédé selon l'invention est cependant également applicable avantageusement, si on le souhaite, à des compositions déjà purifiées par cristallisation en milieu solvant ou dans l'eau et qui, pour l'occasion, sont généralement remises en solution afin d'être traitées conformément à l'invention.

La composition soumise au procédé de purification selon l'invention peut avantageusement consister en une composition d'isohexide, à savoir une composition, quelles que soient son origine, sa nature, sa forme de présentation, et sa composition, contenant un isohexide en mélange ou non avec un ou plusieurs autres isohexide(s), ledit isohexide étant en tout état de cause l'espèce la plus présente et généralement majoritaire, au sein de la MS contenue dans ladite composition.

Selon la nature de l'isohexide ainsi prédominant pondéralement, il peut s'agir en particulier d'une composition d'isosorbide, d'isomannide, d'isoidide ou d'isogalactide.

Selon une variante préférentielle, la composition soumise au procédé de purification selon l'invention consiste en une composition d'isosorbide.

La société Demanderesse a trouvé que de façon remarquable, le procédé de purification tel que revendiqué, à savoir associant la mise en oeuvre successive d'un moyen d'échange ionique puis de décoloration, permettait d'obtenir des compositions améliorées en terme de stabilité.

Par « stabilité », on entend notamment la stabilité dans le temps de la composition en termes d'évolution du pH, de la conductivité et/ou de la teneur en certaines impuretés. Celles-ci concernent notamment l'acide formique et, de manière générale, les espèces ioniques, l'ensemble de ces produits n'ayant pas été spécifiquement étudiés dans l'art antérieur susmentionné. La Société Demanderesse pense qu'en toute hypothèse, tout ou partie de ces produits pourraient jouer le rôle de « promoteurs » et/ou de « révélateurs » de l'instabilité des compositions ici envisagées.

Ce résultat est d'autant plus surprenant que les travaux menés par la Demanderesse ont montré que les moyens de décoloration et d'échange ionique mis en oeuvre non seulement de manière individuelle mais également associés selon un ordre inverse (décoloration puis échange ionique), ne permettaient aucunement d'atteindre les mêmes performances, notamment en termes de stabilité de la composition purifiée résultante.

Il a par exemple été observé que le traitement d'une composition d'isosorbide préalablement passée sur une colonne de charbon actif granulaire, par une succession de résines cationique forte puis anionique forte avait, en définitive, un effet déstabilisant sur la composition d'isosorbide décolorée ainsi obtenue. La Demanderesse a trouvé en particulier qu'une telle mise en oeuvre, non conforme à l'invention, avait pour effet défavorable d'augmenter la propension de la composition d'isosorbide 1) à s'acidifier globalement (mesure du pH) et notamment 2) à générer de l'acide formique mais aussi 3) à générer des espèces ioniques (mesure globale de la conductivité), lors d'un stockage même de faible durée (2 jours) à température relativement peu élevée (60°C).

Le fait de mettre en oeuvre un second moyen de décoloration, en l'occurrence du charbon actif pulvérulent, avant le traitement sur résines n'a pas permis de diminuer significativement cette propension.

A l'inverse, le fait de mettre en oeuvre le même charbon actif après le même traitement sur résines et ce, conformément à la présente invention, a permis curieusement d'obtenir une composition d'isosorbide qui, lors du même traitement de stockage, a montré une propension beaucoup moins marquée à s'acidifier et à générer de l'acide formique et des espèces ioniques.

En suite de quoi, on dispose désormais d'un moyen, simple et efficace, de préparation de compositions améliorées d'isohexides et autres sucres hydrogénés déshydratés (sorbitan, mannitan, iditan, galactitan, xylitan, ribitan ou érythritan), en particulier de compositions améliorées d'isosorbide et d'isomannide.

Ce moyen permet notamment l'obtention de compositions d'isosorbide ou d'isomannide présentant :
- une pureté en isosorbide ou isomannide au moins égale à 98,5 %, de préférence au moins égale à 99,0 %, exprimée en poids sec par rapport au poids sec total de ladite composition, et
- une teneur en acide formique, sous forme libre et/ou de sels, au plus égale à 0,01 %, exprimée également en poids sec par rapport au poids sec total de ladite composition,
- de préférence, une conductivité au plus égale à 20 microsiemens par centimètre (20 µS/cm).

Ladite conductivité est mesurée selon un test A consistant à ajuster, si besoin, la matière sèche (MS) de la composition d'isosorbide à une valeur de 5 % en diluant ladite composition dans de l'eau distillée ou, inversement, en la concentrant sous vide puis en mesurant la conductivité de la composition à 5 % ainsi obtenue.

De façon remarquable, ce moyen permet, notamment en association avec une étape antérieure ou postérieure de cristallisation, l'obtention de compositions d'isosorbide ainsi caractérisées et présentant une pureté au moins égale à 99,5 %, de préférence au moins égale à 99,6 % et par exemple de l'ordre de 99,8 % comme il sera exemplifié par ailleurs.

Le procédé selon l'invention permet également l'obtention de compositions d'isosorbide ou d'isomannide présentant :
- une pureté en isosorbide ou isomannide au moins égale à 98,5 %, de préférence au moins égale à 99,0 %,
- une teneur totale en monoanhydro pentitols au plus égale à 0,1% de préférence au plus égale à 0,07 %, et
- une teneur totale en mono déoxy- et di déoxy- mono anhydro hexitols au plus égale à 0,1 %, de préférence au plus égale à 0,08 %, ces pourcentages étant exprimés en poids sec par rapport au poids sec total de ladite composition.

Comme indiqué, ce procédé permet l'obtention de compositions d'isosorbide ainsi caractérisées et présentant une pureté au moins égale à 99,5 %, de préférence au moins égale à 99,6 %.

La teneur en isosorbide et autres sucres hydrogénés déshydratés est mesurée classiquement par chromatographie en phase gazeuse (CPG).

La teneur en acide formique est mesurée classiquement par chromatographie liquide haute performance (HPLC).

Du fait qu'elles sont susceptibles d'être obtenues conformément à l'invention sans faire obligatoirement appel à une étape d) de cristallisation en milieu solvant organique, et/ou en faisant intervenir une étape finale de séchage, les compositions d'isosorbide ou d'isomannide ainsi caractérisées peuvent être avantageusement exemptes de traces de tout solvant organique pour certaines applications.

Les compositions d'isosorbide et autres produits de déshydratation interne de sucres hydrogénés comme l'isomannide telles qu'obtenues selon l'invention peuvent se présenter sous diverses formes liquides ou solides et notamment de distillats purifiés, de massés ou de compositions de cristaux bien individualisés. Les formes solides peuvent présenter en outre une couleur blanche et une teneur en eau au plus égale à 1 %, de préférence au plus égale à 0,6 % et en particulier comprise entre 0,10 % et 0,55 %.

Compte tenu de leurs caractéristiques de pureté, de stabilité et/ou de couleur, ces compositions peuvent être utilisées avantageusement dans de nombreuses industries et notamment comme intermédiaire de synthèse, comonomère (y compris allongeur de chaînes), agent solvant, agent plastifiant, agent lubrifiant, agent de charge, édulcorant et/ou principe actif, dans la préparation de produits ou de mélanges, polymériques ou non, biodégradables ou non, destinés aux industries chimiques, pharmaceutiques, cosmétologiques ou alimentaires.

La présente invention va être décrite de façon encore plus détaillée à l'aide des exemples qui suivent et qui ne sont aucunement limitatifs.

### EXEMPLE 1

Dans un réacteur agité double enveloppe, on introduit 1 kg d'une solution de sorbitol à 70 % de MS commercialisée par la Demanderesse sous l'appellation « NEOSORB® 70/02 » et 7 g d'acide sulfurique concentré. Le mélange obtenu est chauffé sous vide (pression d'environ 100 mbars) pendant 5 heures de façon à éliminer l'eau contenue dans le milieu réactionnel initial et celle provenant de la réaction de déshydratation du sorbitol.

Le brut réactionnel est ensuite refroidi vers 100°C puis neutralisé avec 11,4 g d'une solution de soude à 50 %. La composition d'isosorbide ainsi neutralisée est ensuite distillée sous vide (pression inférieure à 50 mbars).

Le distillat brut d'isosorbide, légèrement coloré (couleur jaune claire) est ensuite mis en solution dans de l'eau distillée de façon à obtenir une solution à 40 % de MS.

Cette solution est ensuite percolée sur une colonne de charbon actif granulaire de type « CECA DC 50 » à une vitesse de 0,5 BV/h (« Bed Volume » ou « Volume de lit » / heure). La composition d'isosorbide décolorée ainsi obtenue est ensuite passée, à une vitesse de 2 BV/h, successivement sur une colonne de résine cationique forte de type « PUROLITE C 150 S » puis une colonne de résine anionique forte de type « AMBERLITE IRA 910 ».

Cette solution est ensuite traitée par du charbon actif en poudre de type « NORIT SX+ » à 20°C pendant 1 heure. Le charbon actif est mis en oeuvre à raison de 5 % exprimé en poids sec / poids sec de solution.

Après filtration, la solution d'isosorbide est concentrée sous vide. La masse fondue obtenue cristallise au refroidissement sous forme d'un « massé » de gros cristaux que l'on broye ensuite pour obtenir une poudre de couleur blanche présentant une humidité de 0,3 % et donc une MS de 99,7 %.

Cette composition d'isosorbide, obtenue conformément à l'invention, présente les caractéristiques ci-après, les pourcentages étant exprimés par rapport au poids total de ladite composition.

| | |
|---|---|
| eau | 0,3 |
| isosorbide | 98,8 % |
| isomannide | 0,3 % |
| autres dianhydro hexitols | 0,3 % |
| mono anhydro pentitols | < 0,025 % |
| déoxy hexitols | < 0,070 % |
| acide formique | < 0,0005 % |
| pH* | 6,6 |
| Conductivité* | < 20 µS/cm |

| | |
|---|---|
| * mesuré(e) après dilution de la composition à une MS de 5 %. | |

La composition obtenue, conforme à l'invention, présente donc une pureté en isosorbide de 98,8 / 99,7, soit d'environ 99,1 %.

20 g de ce massé d'isosorbide sont introduits dans un pot en plastique bouché, lui même stocké dans une étude à 60°C. Après 2 jours de stockage dans ces conditions, on note que cette composition a légèrement évolué en termes de pH (baisse de 0,6 unité de pH) mais qu'en particulier sa pureté en isosorbide, sa concentration en acide formique et sa conductivité n'ont pas significativement évolué, ces deux dernières caractéristiques restant à un niveau très bas (respectivement < 0,005 % et < 50 µS/cm).

### EXEMPLE 2 (COMPARATIF)

Dans ces essais, le distillat brut d'isosorbide tel qu'obtenu lors de l'EXEMPLE 1 est purifié selon différentes variantes non conformes à l'invention, à savoir respectivement :
- ESSAI 1 : le distillat est traité uniquement sur du charbon actif granulaire avant d'être cristallisé sous forme de massé,
- ESSAI 2 : le distillat est traité uniquement sur résines (cationique forte puis anionique forte) avant d'être cristallisé (massé),
- ESSAI 3 : le distillat est traité d'abord sur charbon actif puis sur résines (cationique forte puis anionique forte) avant d'être cristallisé (massé),
- ESSAI 4 : le distillat est traité successivement sur du charbon actif granulaire puis avec du charbon actif pulvérulent avant d'être filtré puis traité sur résines et cristallisé (massé).

On constate que l'ensemble des compositions d'isosorbide telles que résultant directement de ces procédés, présentent globalement :
- un pH très légèrement ou significativement inférieur à celui du massé obtenu dans l'EXEMPLE 1, à savoir un pH se situant entre 5,6 (ESSAI 2) et 6,5 environ (ESSAIS 3 et 4),
- une conductivité inférieure à 20 µS/cm (ESSAIS 2 à 4), voire légèrement supérieure (23 µS/cm - ESSAI 1), et
- une proportion d'acide formique inférieure à 0,0005 % ou 5 ppm (ESSAIS 2 à 4), voire légèrement supérieure (0,0015 % ou 15 ppm - ESSAI 1).

Cependant, on constate que dans des conditions de stockage identiques à celles décrites pour l'EXEMPLE 1, l'ensemble de ces compositions montrent :
- une baisse de pH au moins triple de celle observée dans le cadre de l'EXEMPLE 1, et notamment 4 fois (ESSAI 4) à 5 fois (ESSAI 3) supérieure à cette dernière,
- une génèse d'acide formique beaucoup plus significative que dans le cadre de l'EXEMPLE 1, le taux d'acide formique dépassant toujours la valeur de 0,01 % et atteignant des valeurs supérieures à 0,02 % (ESSAI 4), voire supérieures à 0,04 % (ESSAI 3) et même à 0,08 % (ESSAI 2).

En outre, les compositions issues des ESSAIS 2 et 3 montrent une propension très marquée à générer des espèces ioniques au stockage puisque, après 2 jours seulement, leur conductivité atteint la valeur de 40 µS/cm (ESSAI 3), voire dépasse la valeur de 80 µS/cm (ESSAI 2).

En suite de quoi, de manière très surprenante le procédé conforme à l'invention tel qu'imaginé par la Demanderesse dans l'EXEMPLE 1 et envisageant la mise en oeuvre d'un moyen de décoloration après un moyen d'échange ionique, permet d'obtenir des compositions, en l'occurrence d'isosorbide, beaucoup plus stables que celles obtenues selon l'ensemble des procédés décrits dans le présent

### EXEMPLE 2.

Cette amélioration par la mise en oeuvre de ces deux moyens spécifiques selon un ordre particulier est d'autant plus inattendue si l'on considère les effets obtenus respectivement soit avec chacun de ces moyens pris isolément (ESSAIS 1 et 2) soit avec ces moyens associés mais dans un ordre inverse (ESSAIS 3 et 4).

### EXEMPLE 3

Dans ces essais, conformes à l'invention, un distillat brut d'isosorbide est obtenu puis traité de la même façon qu'à l'EXEMPLE 1, à l'exception de certaines variantes, à savoir respectivement :
- ESSAI 5 : le massé tel qu'obtenu à l'EXEMPLE 1 est remis en solution, à chaud, dans du méthanol. La solution résultante à 75 % de MS, est refroidie jusqu'à une température de - 15°C environ et se transforme en une composition de cristaux mieux individualisés que ceux du massé initial,
- ESSAI 6 : la composition de sorbitol utilisée comme matière première en vue de l'étape de déshydratation proprement dite a été préalablement purifiée par chromatographie au moyen d'une colonne PUROLITE de type « PCR 432 » sous forme calcium,
- ESSAI 7 : l'étape de distillation se fait en présence de 0,5 % de Na B H4 (sec/sec),
- ESSAI 8 : la solution à 40 % MS soumise au traitement sur charbon actif granulaire puis, conformément à l'invention, sur résines cationique et anionique puis sur charbon actif pulvérulent, résulte de la redissolution dans l'eau de cristaux d'isosorbide, ceux-ci ayant été obtenus classiquement par cristallisation dans le méthanol d'un massé d'isosorbide lui-même obtenu par distillation.

L'ensemble des compositions obtenues, conformes à l'invention, présentent une teneur en eau comprise entre 0,1 % (ESSAI 8) et 0,5 % (ESSAI 5) ainsi qu'une pureté encore (légèrement) supérieure à celle du produit issu de l'EXEMPLE 1. Cette pureté en isosorbide est comprise entre 99,2 % (ESSAI 7) et 99,6 % (ESSAIS 5, 6 et 8).

Il est d'ailleurs remarquable qu'un tel niveau de pureté (99,6 %) puisse être obtenu, en particulier, en l'absence de toute étape de cristallisation conformément à l'ESSAI 6.

Ce produit et ceux résultant des ESSAIS 5, 7 et 8 présentent par ailleurs une bonne stabilité au stockage et des taux d'impuretés particulières conformes à la présente invention.

### EXEMPLE 4

Le distillat brut, tel qu'obtenu dans l'EXEMPLE 1, est mis en solution dans le 2-propanol, à une température de 60°C, de façon à obtenir une solution à 75 % de matière sèche (MS). Cette solution est ensuite refroidie lentement, en l'espace de 5 heures, à une température de 10°C. Une amorce d'isosorbide recristallisé est ajoutée à 40°C.

Les cristaux sont ensuite essorés dans une essoreuse et lavés avec un peu de 2-propanol.

Après séchage sous vide, les cristaux sont remis en solution dans l'eau de façon à obtenir une MS de 40 %.

Cette solution est percolée sur une colonne de charbon actif granulaire (CPG 12-40) à une vitesse de 0,5 BV/h. La composition d'isosorbide, ainsi obtenue, est ensuite passée, à une vitesse de 2 BV/h, successivement sur une colonne de résine cationique forte puis une colonne de résine anionique forte comme celles décrites dans l'EXEMPLE 1 de ce brevet.

La solution est ensuite traitée par du charbon actif en poudre de type NORIT SX⁺ à 20°C pendant 1 heure. Le charbon actif est mis en oeuvre à raison de 0.2 % exprimé en poids sec / poids sec de solution.

Après filtration, la solution d'isosorbide est concentrée sous vide. La masse fondue obtenue cristallise au refroidissement sous forme d'un massé qui est ensuite broyé pour obtenir une poudre blanche présentant une humidité de 0.2 %.

Cette composition d'isosorbide, obtenue conformément à l'invention, présente les caractéristiques ci-après, les pourcentages étant exprimés par rapport au poids total de ladite composition.

| | |
|---|---|
| Eau | 0,2 % |
| Isosorbide | 99,6 % |
| Isomannide | Non détecté |
| Autres dianhydrohexitols | 0,03 % |
| Mono anhydropentitols | Non détecté |
| Déoxyhexitols | Non détecté |
| Acide formique | < 0,0005 % |
| PH | 6,5 |
| Conductivité | < 20 µS/cm |

Sa pureté en isosorbide est donc de 99,6 / 99,8, soit d'environ 99,8 %.

20 g de ce massé d'isosorbide sont introduits dans un pot en plastique bouché, lui-même stocké dans un étuve à 60°C. Après 14 jours de stockage, on note que sa pureté en isosorbide et sa concentration en acide formique n'ont pas évolué.

### EXEMPLE 5

Dans un réacteur agité double enveloppe, on introduit 700 g de mannitol, 300 g d'eau et 21 g d'acide sulfurique concentré. Le mélange obtenu est chauffé sous vide (40 mbars) pendant 8 heures de façon à éliminer l'eau contenue dans le milieu réactionnel et celle provenant de la réaction de déshydratation du mannitol.

Le brut réactionnel est neutralisé avec une solution de soude à 50 %. La composition d'isomannide est ensuite distillée sous vide.

Le distillat brut est mis en solution dans l'eau de façon à obtenir une matière sèche de 40 %.

Cette solution est percolée sur une colonne de charbon actif granulaire (CPG 12-40) à une vitesse de 0,5 BV/h. La composition d'isomannide, ainsi obtenue, est ensuite passée, à une vitesse de 2 BV/h, successivement sur une colonne de résine cationique forte puis une colonne de résine anionique forte comme celles décrites dans l'exemple 1.

La solution est ensuite traitée par du charbon actif en poudre de type NORIT SX+ à 20°C pendant 1 heure. Le charbon actif est mis en oeuvre à raison de 5 % exprimé en poids sec / poids sec de solution.

Après filtration, la solution d'isomannide est concentrée sous vide. La masse fondue obtenue cristallise au refroidissement sous forme d'un massé qui est ensuite broyé pour obtenir une poudre blanche présentant une humidité à 0,3 %.

Cette composition d'isomannide, obtenue conformément à l'invention, présente les caractéristiques ci-après, les pourcentages étant exprimés par rapport au poids total de ladite composition.

| | |
|---|---|
| Eau | 0,5 % |
| Isomannide | 98,0 % |
| Isosorbide | 0,9 % |
| Autres dianhydrohexitols | 0,3 % |
| Acide formique | < 0,0005 % |
| PH | 6,5 |
| Conductivité | < 20 µS/cm |

Sa pureté en isomannide est donc de 98 / 99,5 %, soit d'environ 98,5 %.

20 g de ce massé d'isomannide sont introduits dans un pot en plastique bouché, lui-même stocké dans un étuve à 60°C. Après 14 jours de stockage, on note que sa pureté en isosorbide et sa concentration en acide formique n'ont pas évolué.

## Revendications

1. Procédé de purification d'une composition contenant au moins un produit de déshydratation interne d'un sucre hydrogéné, **caractérisé en ce qu'**il comprend :
a) une étape éventuelle au cours de laquelle ladite composition est traitée, après remise en solution ou non, avec au moins un moyen de décoloration,
b) une étape subséquente au cours de laquelle la composition, éventuellement traitée avec un moyen de décoloration, est traitée avec au moins un moyen d'échange ionique,
c) une étape subséquente au cours de laquelle la composition résultante est traitée avec au moins un moyen de décoloration.

2. Procédé selon la revendication 1, **caractérisé en ce qu'**il comprend :
a) Une étape au cours de laquelle ladite composition, remise en solution ou non, est traitée avec du charbon actif, de préférence sous forme granulaire,
b) Une étape subséquente au cours de laquelle la composition résultante est traitée avec au moins un moyen d'échange ionique,
c) Une étape subséquente au cours de laquelle la composition résultante est traitée avec du charbon actif, de préférence sous forme pulvérulente.

3. Procédé selon l'une des revendications 1 ou 2, **caractérisé en ce que** le moyen d'échange ionique comprend au moins une résine anionique, de préférence au moins une résine anionique forte, et au moins une résine cationique, de préférence au moins une résine cationique forte.

4. Procédé selon l'une quelconque des revendications 1 à 3, **caractérisé en ce qu'**il comprend une étape d) au cours de laquelle la composition résultant de l'étape c) est soumise, après filtration éventuelle, à un traitement de concentration en vue d'obtenir un massé, puis éventuellement à un traitement de cristallisation, en particulier en milieu solvant, et/ou de séchage.

5. Procédé selon l'une quelconque des revendications 1 à 4, **caractérisé en ce que** la composition contenant au moins un produit de déshydratation interne d'un sucre hydrogéné consiste en une composition d'isohexide, de préférence choisie dans le groupe comprenant les compositions d'isosorbide, d'isomannide, d'isoidide et d'isogalactide et plus préférentiellement encore consiste en une composition d'isosorbide ou d'isomannide.

## Patentansprüche

1. Verfahren zur Reinigung einer Zusammensetzung, die mindestens ein inneres Dehydratationsprodukt eines hydrierten Zuckers enthält, **dadurch gekennzeichnet, dass** es umfasst:
a) einen wahlweisen Schritt, im Verlauf dessen die Zusammensetzung, nach einem Versetzen in Lösung oder nicht, mit mindestens einem Entfärbungsmittel behandelt wird,
b) einen anschließenden Schritt, im Verlauf dessen die Zusammensetzung, die gegebenenfalls mit einem Entfärbungsmittel behandelt wurde, mit mindestens einem Ionenaustauschmittel behandelt wird,
c) einem anschließenden Schritt, im Verlauf dessen die resultierende Zusammensetzung mit mindestens einem Entfärbungsmittel behandelt wird.

2. Verfahren gemäß Anspruch 1, **dadurch gekennzeichnet, dass** es umfasst:
a) einen Schritt, im Verlauf dessen die Zusammensetzung, die entweder in Lösung versetzt wird oder nicht, mit Aktivkohle, vorzugsweise in granulärer Form, behandelt wird,
b) einem anschließenden Schritt, im Verlauf dessen die resultierende Zusammensetzung mit mindestens einem Ionenaustauschmittel behandelt wird,
c) einem anschließenden Schritt, im Verlauf dessen die resultierende Zusammensetzung mit Aktivkohle, vorzugsweise in Pulverform, behandelt wird.

3. Verfahren gemäß einem der Ansprüche 1 oder 2, **dadurch gekennzeichnet, dass** das Ionenaustauschmittel mindestens ein anionisches Harz, vorzugsweise mindestens ein starkes anionisches Harz, und mindestens ein kationisches Harz, vorzugsweise mindestens ein starkes kationisches Harz, umfasst.

4. Verfahren gemäß irgendeinem der Ansprüche 1 bis 3, **dadurch gekennzeichnet, dass** es einen Schritt d) umfasst, im Verlauf dessen die aus dem Schritt c) resultierende Zusammensetzung, gegebenenfalls nach Filtration, einer Konzentrationsbehandlung zum Zweck des Erhaltens einer Masse, dann gegebenenfalls einer Kristallisationsbehandlung, insbesondere aus einem Lösungsmedium, und/oder einer Trocknung unterzogen wird.

5. Verfahren gemäß irgendeine der Ansprüche 1 bis 4, **dadurch gekennzeichnet, dass** die Zusammensetzung, die mindestens ein inneres Dehydratationsprodukt eines hydrogenierten Zuckers enthält, aus einer Isohexid-Zusammensetzung besteht, vorzugsweise ausgewählt aus der Gruppe, die Isosorbid-, Isomannid-, Isoidid- und Isogalactid-Zusammensetzungen umfasst, und weiter bevorzugt bestehend aus einer Isosorbid- oder Isomannid-Zusammensetzung.

## Claims

1. A method for purifying a composition containing at least one internal dehydration product of a hydrogenated sugar, **characterized in that** it comprises:
a) an optional step during which said composition is treated, after redissolving or not, with at least one decolorizing means,
b) a subsequent step during which the composition, optionally treated with a decolorizing means, is treated with at least one ion-exchange means, and
c) a subsequent step during which the resulting composition is treated with at least one decolorizing means.

2. The method as claimed in claim 1, **characterized in that** it comprises:
a) a step during which the composition, which has been redissolved or not, is treated with activated charcoal, preferably in granular form,
b) a subsequent step during which the resulting composition is treated with at least one ion-exchange means,
c) a subsequent step during which the resulting composition is treated with activated charcoal, preferably in pulverulent form.

3. The method as claimed in either of claims 1 and 2, **characterized in that** the ion-exchange means comprises at least one anionic resin, preferably at least one strong anionic resin, and at least one cationic resin, preferably at least one strong cationic resin.

4. The method as claimed in any one of claims 1 to 3, **characterized in that** it comprises a step d) during which the composition resulting from step c) is subjected, after optional filtration, to a concentration treatment in order to obtain a massecuite product optionally followed by crystallization treatment, in particular from an organic solvent medium and/or drying.

5. The method as claimed in any one of claims 1 to 4, **characterized in that** the composition containing at least one internal dehydration product of a hydrogenated sugar consists of an isohexide composition, preferably chosen from the group comprising isosorbide, isomannide, isoidide and isogalactide compositions and still more preferably consists of an isosorbide or isomannide composition.
